# EUROPÄISCHE PATENTSCHRIFT

(11) **EP 1 084 248 B1**
(45) Veröffentlichungstag und Bekanntmachung des Hinweises auf die Patenterteilung: **26.07.2006**
(21) Anmeldenummer: 99925023.6
(22) Anmeldetag: 21.05.1999
(51) Int. Cl.: C12N 15/17, C07K 14/62, A61K 38/28

(54) **NEUE INSULINANALOGA MIT ERHÖHTER ZINKBINDUNG**
NOVEL INSULIN ANALOGS WITH ENHANCED ZINC BINDING
NOUVEAUX ANALOGUES DE L'INSULINE PRESENTANT UN POUVOIR DE LIAISON ACCRU AVEC LE ZINC

(30) Priorität: 06.06.1998 DE 19825447
(43) Veröffentlichungstag der Anmeldung: 21.03.2001
(73) Patentinhaber: Sanofi-Aventis Deutschland GmbH, 65929 Frankfurt am Main (DE)
(72) Erfinder: ERTL, Johann, D-65817 Eppstein (DE); HABERMANN, Paul, D-65817 Eppstein (DE); GEISEN, Karl, D-60318 Frankfurt (DE); SEIPKE, Gerhard, D-65719 Hofheim (DE); WOLLMER, Axel, D-52074 Aachen (DE)
(86) Internationale Anmeldenummer: PCT/EP1999/003490
(87) Internationale Veröffentlichungsnummer: WO 1999/064598

(56) Entgegenhaltungen:
- EP-A- 0 214 826
- EP-A- 0 668 292
- EP-A- 0 821 006
- WO-A-95/00550
- WO-A-95/07931

### SEQUENZPROTOKOLL

(1) ALLGEMEINE INFORMATION:
   (i) ANMELDER:
      (A) NAME: Hoechst Marion Roussel Deutschland GmbH
      (B) STRASSE: -
      (C) ORT: Frankfurt
      (D) BUNDESLAND: -
      (E) LAND: Germany
      (F) POSTLEITZAHL: 65926
      (G) TELEPHON: 069-305-5307
      (H) TELEFAX: 069-357175
      (I) TELEX: -
   (ii) ANMELDETITEL: Neue Insulinderivate zur Behandlung von Diabetes mellitus
   (iii) ANZAHL DER SEQUENZEN: 11
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 21 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..21
   (xi) SEQUENZBESCHREIBUNG: SEQ ID NO: 1:
   Gly Ile Val Glu Gln Cys Cys Thr Ser Ile Cys Ser Leu Tyr Gln Leu
   1               5                   10                  15
   Glu Asn Tyr Cys Asn
               20
(2) INFORMATION ZU SEQ ID NO: 2:
   (i) SEQUENZ CHARAKTERISTIKA:
      (A) LÄNGE: 30 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..30
   (xi) SEQUENZBESCHREIBUNG: SEQ ID NO: 2:
   Phe Val Asn Gln His Leu Cys Gly Ser His Leu Val Glu Ala Leu Tyr
   1               5                   10                  15
   Leu Val Cys Gly Glu Arg Gly Phe Phe Tyr Thr Pro Lys Thr
               20                  25                  30
(2) INFORMATION ZU SEQ ID NO: 3:
   (i) SEQUENZ CHARAKTERISTIKA:
      (A) LÄNGE: 30 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..30
   (xi) SEQUENZBESCHREIBUNG: SEQ ID NO: 3:
   His Phe Val Asn Gln His Leu Cys Gly Ser His Leu Val Glu Ala Leu
   1               5                   10                  15
   Tyr Leu Val Cys Gly Glu Arg Gly Phe Phe Tyr Thr Pro Lys
               20                  25                  30
(2) INFORMATION ZU SEQ ID NO: 4:
   (i) SEQUENZ CHARAKTERISTIKA:
      (A) LÄNGE: 31 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..31
   (xi) SEQUENZBESCHREIBUNG: SEQ ID NO: 4:
   His Phe Val Asn Gln His Leu Cys Gly Ser His Leu Val Glu Ala Leu
   1               5                   10                  15
   Tyr Leu Val Cys Gly Glu Arg Gly Phe Phe Tyr Thr Pro Lys Thr
               20                  25                  30
(2) INFORMATION ZU SEQ ID NO: 5:
   (i) SEQUENZ CHARAKTERISTIKA:
      (A) LÄNGE: 32 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..32
   (xi) SEQUENZBESCHREIBUNG: SEQ ID NO: 5:
   His Ala Phe Val Asn Gln His Leu Cys Gly Ser His Leu Val Glu Ala
   1               5                   10                  15
   Leu Tyr Leu Val Cys Gly Glu Arg Gly Phe Phe Tyr Thr Pro Lys Thr
               20                  25                  30
(2) INFORMATION ZU SEQ ID NO: 6:
   (i) SEQUENZ CHARAKTERISTIKA:
      (A) LÄNGE: 33 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..33
   (xi) SEQUENZBESCHREIBUNG: SEQ ID NO: 6:
   His Ala Ala Phe Val Asn Gln His Leu Cys Gly Ser His Leu Val Glu
   1               5                   10                  15
   Ala Leu Tyr Leu Val Cys Gly Glu Arg Gly Phe Phe Tyr Thr Pro Lys
               20                  25                  30
   Thr
(2) INFORMATION ZU SEQ ID NO: 7:
   (i) SEQUENZ CHARAKTERISTIKA:
      (A) LÄNGE: 98 Aminosäuren
      (B) ART: Aminosäure
      (C) STRANGFORM: Einzel
      (D).TOPOLOGIE: linear
   (ii) ART DES MOLEKÜLS: Protein
   (ix) MERKMALE:
      (A) NAME/SCHLÜSSEL: Protein
      (B) LAGE: 1..98
   (xi) SEQUENZBESCHREIBUNG: SEQ ID NO: 7:
   Met Ala Thr Thr Ser Thr Gly Asn Ser Ala Arg His Phe Val Asn Gln
   1 5 10 15
   His Leu Cys Gly Ser His Leu Val Glu Ala Leu Tyr Leu Val Cys Gly
   20 25 30
   Glu Arg Gly Phe Phe Tyr Thr Pro Lys Thr Arg Arg Glu Ala Glu Asp
   35 40 45
   Pro Gln Val Gly Gln Val Glu Leu Gly Gly Gly Pro Gly Ala Gly Ser
   50 55 60
   Leu Gln Pro Leu Ala Leu Glu Gly Ser Leu Gln Lys Arg Gly Ile Val
   65 70 75 80
   Glu Gln Cys Cys Thr Ser Ile Cys Ser Leu Tyr Gln Leu Glu Asn Tyr
   85 90 95
   Cys Asn
(2) INFORMATION ZU SEQ ID NO: 8:
   (i) SEQUENZ CHARAKTERISTIKA:
      (A) LÄNGE: 99 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..99
   (xi) SEQUENZBESCHREIBUNG: SEQ ID NO: 8:
   Met Ala Thr Thr Ser Thr Gly Asn Ser Ala Arg His Ala Phe Val Asn
   1 5 10 15
   Gln His Leu Cys Gly Ser His Leu Val Glu Ala Leu Tyr Leu Val Cys
   20 25 30
   Gly Glu Arg Gly Phe Phe Tyr Thr Pro Lys Thr Arg Arg Glu Ala Glu
   35 40 45
   Asp Pro Gln Val Gly Gln Val Glu Leu Gly Gly Gly Pro Gly Ala Gly
   50 55 60
   Ser Leu Gln Pro Leu Ala Leu Glu Gly Ser Leu Gln Lys Arg Gly Ile
   65 70 75 80
   Val Glu Gln Cys Cys Thr Ser Ile Cys Ser Leu Tyr Gln Leu Glu Asn
   85 90 95
   Tyr Cys Asn
(2) INFORMATION ZU SEQ ID NO: 9:
   (i) SEQUENZ CHARAKTERISTIKA:
      (A) LÄNGE: 100 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..100
   (xi) SEQUENZBESCHREIBUNG: SEQ ID NO: 9:
   Met Ala Thr Thr Ser Thr Gly Asn Ser Ala Arg His Ala Ala Phe Val
   1               5                   10                  15
   Asn Gln His Leu Cys Gly Ser His Leu Val Glu Ala Leu Tyr Leu Val
               20                  25                  30
   Cys Gly Glu Arg Gly Phe Phe Tyr Thr Pro Lys Thr Arg Arg Glu Ala
           35                  40                  45
   Glu Asp Pro Gln Val Gly Gln Val Glu Leu Gly Gly Gly Pro Gly Ala
       50                  55                  60
   Gly Ser Leu Gln Pro Leu Ala Leu Glu Gly Ser Leu Gln Lys Arg Gly
   65                  70                  75                  80
   Ile Val Glu Gln Cys Cys Thr Ser Ile Cys Ser Leu Tyr Gln Leu Glu
                   85                  90                  95
   Asn Tyr Cys Asn
               100
(2) INFORMATION ZU SEQ ID NO: 10:
   (i) SEQUENZ CHARAKTERISTIKA:
      (A) LÄNGE: 30 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..30
   (xi) SEQUENZBESCHREIBUNG: SEQ ID NO: 10:
   Phe Val His Gln His Leu Cys Gly Ser His Leu Val Glu Ala Leu Tyr
   1               5                   10                      15
   Leu Val Cys Gly Glu Arg Gly Phe Phe Tyr Thr Pro Lys Thr
               20                  25                  30
(2) INFORMATION ZU SEQ ID NO: 11
   (i) SEQUENZ CHARAKTERISTIKA:
      (A) LÄNGE: 30 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..30
   (xi) SEQUENZBESCHREIBUNG: SEQ ID NO: 11
   Phe Val Asp Gln His Leu Cys Gly Ser His Leu Val Glu Ala Leu Tyr
   1               5                   10                      15
   Leu Val Cys Gly Glu Arg Gly Phe Phe Tyr Thr Pro Lys Thr
               20                  25                  30

## Beschreibung

Die vorliegende Erfindung betrifft Insulinanaloga, welche ein erhöhtes Zinkbindungsvermögen aufweisen, sowie stabile Zinkkomplexe derselben, die im Vergleich zu Humaninsulin ein verzögertes Wirkprofil aufweisen, ein Verfahren zu deren Herstellung und deren Verwendung insbesondere in pharmazeutischen Zubereitungen zur Therapie des Diabetes mellitus vom Typ I wie auch Typ II.

Weltweit leiden etwa 120 Mio. Menschen an Diabetes mellitus. Darunter sind etwa 12 Mio. Typ I-Diabetiker, für die die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenem Stadium, die Behandlung mit Insulin, gegebenenfalls in Kombination mit einem oralen Antidiabetikum, als günstigste Therapieform angesehen.

Beim Gesunden ist die Insulinfreisetzung durch den Pankreas strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subcutane Applikation von Insulin erreicht in der Regel die oben beschriebene Qualität der physiologischen Regulation der Blutglucose nicht annähernd. Häufig kommt es zu Entgleisungen der Blutglucose nach oben oder unten, die in ihren schwersten Formen lebensbedrohlich sein können. Daneben stellen jedoch auch über Jahre erhöhte Blutglucosespiegel ohne anfängliche Symptome ein erhebliches Gesundheitsrisiko dar. Die großangelegte DCCT-Studie in den USA (The Diabetes Control and Complications Trial Research Group (1993) N, Engl. J. Med. 329, 977-986) wies eindeutig nach, daß chronisch erhöhte Blutglucosespiegel wesentlich für die Entwicklung diabetischer Spätschäden verantwortlich sind. Diabetische Spätschäden sind mikro- und makrovaskuläre Schädigungen, die sich u. U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, daß eine verbesserte Therapie des Diabetes in erster Linie darauf abzielen muß, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Die derzeit verfügbaren Basalinsuline erfüllen diese Anforderung nur unzulänglich. Speziell die häufig verwendeten NPH-Insuline weisen ein zu stark ausgeprägtes Wirkungsmaximum auf und haben eine zu kurze Gesamtwirkung. Dies birgt bei abendlicher Applikation die Gefahr einer nächtlichen Hypoglykämie und einer morgendlichen Hyperglykämie.

In der Internationalen Patentanmeldung WO95/07931 werden verzögert wirkende Insulinderivate beschrieben, die auch als Zink-Komplexe vorliegen können.

In der internationalen Patentanmeldung WO95/00550 werden Insulinkristalle beschrieben, die Protamin enthalten und wobei bei dem Insulin in Position B28 der Aminosäurerest Asparaginsäure (Asp^{B28}) vorliegt.

In der europäischen Patentanmeldung EP 0 214 826 A2 sind neue, schnellwirksame Insulinanaloga beschrieben, die eine geringe Tendenz zur Selbstassoziierung in Form von Dimeren, Tetrameren, Hexameren oder Polymeren haben.

Aus der EP 0 821 006 sind Insulinanaloga mit erhöhtem Zinkbindungsvermögen bekannt, die in Verbindung mit Zink ein gegenüber Humaninsulin verzögertes Wirkprofil aufweisen. Diese Analoga unterscheiden sich von Humaninsulin im wesentlichen durch Variation der Aminosäure in Position A21 der A-Kette und durch Addition eines Histidinrestes oder eines Peptides mit 2 bis 35 Aminosäureresten, welches 1 bis 5 Histidinreste enthält, an Position B30 der B-Kette.

Die Aufgabe der vorliegenden Erfindung ist es, weitere Insulinanaloga (Analoga von humanem oder tierischem Insulin) bereitzustellen, die ein erhöhtes Zinkbindungsvermögen aufweisen, einen stabilen Komplex, enthaltend ein Hexamer des Insulinanalogons und Zink, bilden und in geeigneter Zubereitung bei subcutaner Injektion infolge des im Vergleich zu Humaninsulin verzögerten Wirkprofils eine verbesserte Therapie des Diabetes mellitus vom Typ I wie auch Typ II ermöglichen.

Insulinanaloga leiten sich von natürlich vorkommenden Insulinen, nämlich Humaninsulin (s. SEQ ID NO: 1: A-Kette von Humaninsulin und SEQ ID NO: 2: B-Kette von Humaninsulin) oder tierischen Insulinen, durch Substitution oder Fehlen wenigstens eines natürlich auftretenden Aminosäurerestes und/oder Addition wenigstens eines Aminosäurerestes an A- und/oder B-Kette des natürlich vorkommenden Insulins ab.

Die Aufgabe wird gelöst durch
1. ein Insulinanalogon oder ein physiologisch verträgliches Salz desselben der Formel I 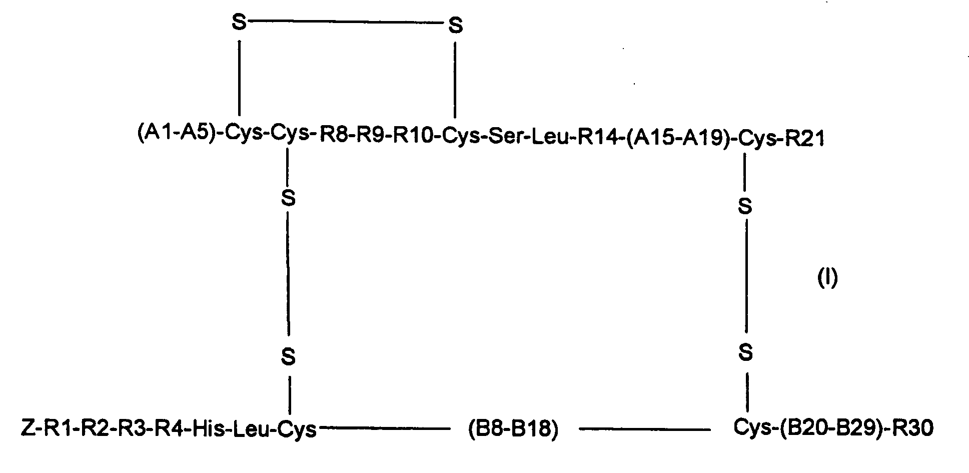 worin bedeuten

| | |
|---|---|
| (A1-A5) | die Aminosäurereste in den Positionen A1 bis A5 der A-Kette von Humaninsulin (vgl. SEQ ID NO: 1) oder tierischem Insulin, |
| (A15-A19) | die Aminosäurereste in den Positionen A15 bis A19 der A-Kette von Humaninsulin (vgl. SEQ ID NO: 1) oder tierischem Insulin, |
| (B8-B18) | die Aminosäurereste in den Positionen B8 bis B18 der B-Kette von Humaninsulin (vgl. SEQ ID NO: 2) oder tierischem Insulin, |

| | |
|---|---|
| (B20-B29) | die Aminosäurereste in den Positionen B20 bis B29 der B-Kette von Humaninsulin (vgl. SEQ ID NO: 2) oder tierischem Insulin, |
| R8 | Thr oder Ala, |
| R9 | Ser oder Gly, |
| R10 | Ile oder Val, |
| R14 | Tyr, His, Asp oder Glu, |
| R21 | Asn, Asp, Gly, Ser, Thr, Ala, Glu oder Gln, |
| R1 | ein beliebiger genetisch kodierbarer Aminosäurerest, abwesend oder ein Wasserstoffatom, |
| R2 | Val, Ala oder Gly, |
| R3 | Asn, His, Glu oder Asp, |
| R4 | Ala, Ser, Thr, Asn, Asp, Gln, Gly oder Glu, |
| R30 | ein beliebiger genetisch kodierbarer Aminosäurerest oder -OH, |
| Z | ein Peptidrest mit 1 bis 4 genetisch kodierbaren Aminosäureresten, enthaltend 1 bis 4 Histidinreste (His), |

mit
der Maßgabe, daß sich das Insulinanalogon oder das physiologisch verträgliche Salz desselben der Formel I nicht allein durch Variation der Aminosäurereste in den Positionen R3 oder R3 in Verbindung mit R21 oder R3 in Verbindung mit R4 in Formel von Humaninsulin (vgl. SEQ ID NO: 1 und SEQ ID NO: 2) unterscheidet.

Vorzugsweise ist das Insulinanalogon oder das physiologisch verträgliche Salz desselben dadurch gekennzeichnet, daß
2. R8 Thr, R9 Ser und R10 Ile bedeuten,
3. R1 Phe, His, Asn, Asp oder Gly bedeutet,
4. R30 Thr, Ala oder Ser bedeutet oder
5. dadurch gekennzeichnet, daß R21 Asn und R1 Phe bedeuten.
6. Eine bevorzugte Ausgestaltung der vorliegenden Erfindung ist ein Insulinanalogon oder ein physiologisch verträgliches Salz desselben der Formel 1, welches dadurch gekennzeichnet ist, daß R2 Val, R3 Asn und R4 Gin bedeuten.

Ferner bevorzugt ist ein Insulinanalogon oder ein physiologisch verträgliches Salz desselben der Formel I, das sich dadurch auszeichnet, daß R14
7. Tyr,
8. His,
9. Asp oder
10. Glu bedeutet.

Ferner bevorzugt ist ein Insulinanalogon oder ein physiologisch verträgliches Salz desselben der Formel I, welches sich dadurch auszeichnet, daß R30
11. Thr,
12. Ala,
13. Ser oder
14. -OH
bedeutet.

Besonders bevorzugt ist ein Insulinanalogon oder ein physiologisch verträgliches Salz desselben der Formel I, das sich dadurch auszeichnet, daß Z
15. His,
16. His-Ala- oder
17. His-Ala-Ala-
bedeutet.

Beispiele für Insulinanaloga gemäß der vorliegenden Erfindung sind
18. ein Insulinanalogon oder ein physiologisch verträgliches Salz desselben der Formel I, das sich dadurch auszeichnet, daß die B-Kette die Sequenz
   His Phe Val Asn Gin His Leu Cys Gly Ser His Leu Val Glu Ala Leu Tyr Leu Val Cys Gly Glu Arg Gly Phe Phe Tyr Thr Pro Lys
   aufweist (SEQ ID NO: 3), beispielsweise das His(B0), des (B30)-Humaninsulin,
19. ein Insulinanalogon oder ein physiologisch verträgliches Salz desselben der Formel I, das sich dadurch auszeichnet, daß die B-Kette die Sequenz
   His Phe Val Asn Gln His Leu Cys Gly Ser His Leu Val Glu Ala Leu Tyr Leu Val Cys Gly Glu Arg Gly Phe Phe Tyr Thr Pro Lys Thr
   aufweist (SEQ ID NO: 4), beispielsweise das His(B0)-Humaninsulin,
20. ein Insulinanalogon oder ein physiologisch verträgliches Salz desselben der Formel I, das sich dadurch auszeichnet, daß die B-Kette die Sequenz
   His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
   aufweist (SEQ ID NO: 5), beispielsweise das His (B-1), Ala (B0)-Humaninsulin oder
21. ein Insulinanalogon oder ein physiologisch verträgliches Salz desselben der Formel I, das sich dadurch auszeichnet, daß die B-Kette die Sequenz
   His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
   aufweist (SEC ID NO: 6), beispielsweise das His(B-2), Ala(B-1), Ala(B0)-Humaninsulin.

Die vorliegende Erfindung betrifft ferner ein Verfahren zur Herstellung des Insulinanalogons oder eines physiologisch verträglichen Salzes desselben gemäß der vorliegenden Erfindung, umfassend die Konstruktion eines replizierbaren Expressionsvehikels, welches eine DNA-Sequenz enthält, die für einen Vorläufer des Insulinanalogons mit der allgemeinen Aminosäuresequenz II kodiert

Met-X²ₘ-(Arg)ₚ-Z-R1-R2-R3-R4-His-Leu-Cys-(B8-B18)-Cys-(B20-B29)-R30-X¹ₙ-Arg-(A1-A5)-Cys-Cys-R8-R9-R10-Cys-Ser-Leu-R14-(A15-A19)-Cys-R21 II,

worin

| | |
|---|---|
| X¹ₙ | eine Peptidkette mit n Aminosäurresten ist, wobei n eine ganze Zahl von 0 bis 34 bedeutet, |
| X²ₘ | eine Peptidkette mit m Aminosäurresten ist, wobei m eine ganze Zahl von 0 bis 20 bedeutet, |
| p | 0, 1 oder 2, |
| R30 | ein beliebiger genetisch kodierbarer Aminosäurerest bedeutet oder abwesend ist und |
| Z | abwesend ist oder ein Peptidrest mit 1 bis 4 genetisch kodierbaren Aminosäureresten, enthaltend 1 bis 4 Histidinreste (His) bedeutet |

und die übrigen Variablen die vorstehend unter Nr. 1 genannten Bedeutungen haben, wobei auch die vorstehend genannten Maßgaben gelten, Expression in einer Wirtszelle und Freisetzung des Insulinanalogons aus dessen Vorläufer mit chemischen und/oder enzymatischen Methoden.

Vorzugsweise ist die Wirtszelle ein Bakterium, besonders bevorzugt Bakterium E. coli.

Vorzugsweise ist die Wirtszelle eine Hefe, besonders bevorzugt Saccharomyces cerevisiae.

Bei der Expression in E. coli bilden die genannten Fusionsproteine (SEQ ID NO: 7 bis 9) in der Regel unlösliche Einschlußkörperchen (inclusion bodies), die nach Zellaufschluß durch Zentrifugation isoliert werden können und unter Verwendung chaotroper Zusätze (z. B. 8 M Harnstoff oder 6 M Guanidiniumchlorid) wieder gelöst werden. Das gelöste Fusionsprotein kann einer Sulfitolyse unterzogen werden, bei der SH-Reste in S-Sulfonate überführt werden (z. B. R.C. Marshall und A.S. Iglis in ,Practical Protein Chemistry - A Handbook', Herausgeber A. Darbre (1986), Seiten 49-53). Dadurch verbessert sich die Löslichkeit des Fusionsproteins und erleichtert die Reinigung beispielsweise mittels Anionenaustauscher- oder Gelpermeationschromatographie.
Die Überführung des derivatisierten Fusionsproteins in Präproinsulin mit nativer räumlicher Struktur und korrekt ausgebildeten Disulfidbrücken (Faltung) erfolgt in verdünnter wäßriger Lösung durch Zusatz einer begrenzten Menge eines SH-Reagens wie Merkaptoethanol, Cystein oder Glutathion und anschließender Luftoxidation. Alternativ kann das gelöste, nicht derivatisierte Fusionsprotein unter ähnlichen Bedingungen auch direkt gefaltet werden (EP-A-0 600 372; EP-A-0 668 292).
Präproinsulin wird anschließend durch limitierte proteolytische Spaltung in biologisch aktives Insulin überführt. Hierfür kann Trypsin verwendet werden, welches die in Formel II mit Met-X²ₘ-(Arg)ₚ bezeichneten Präsequenz entfernt und an der mit X¹ₙ₋Arg bezeichneten Peptidkette spaltet und damit B- und A-Kette trennt. In der Regel beginnt die Sequenz X¹ mit Arg, Arg₂ oder sie ist nicht vorhanden (n=0), so daß nach der Spaltung ein Insulinderivat vorliegt, das mit Arg oder Arg₂ am C-Terminus der B-Kette verlängert ist. Diese Aminosäuren können mit Carboxypeptidase B entfernt werden. Die tryptische Spaltung kann durch Erhöhung der Trypsinkonzentration oder Verlängerung der Reaktionsdauer auch so geführt werden, daß zusätzlich an Lysin(B29) gespalten wird. In diesem Fall entsteht ein des(B30)-Insulinderivat.
Das bei der Spaltung entstandene Insulinanalogon kann durch chromatographische Standardverfahren (z. B. lonenaustauscher- und Reversed-Phase-Chromatographie) gereinigt und zuletzt durch Fällung, Kristallisation oder einfache Gefriertrocknung isoliert werden.

Der Vorläufer des Insulinanalogons hat vorzugsweise die Sequenz
Met Ala Thr Thr Ser Thr Gly Asn Ser Ala Arg
His Phe Val Asn Gin His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gin Val Glu Leu Gly Gly Gly Pro Gly Ala Gly Ser Leu Gin Pro Leu Ala Leu Glu Gly Ser Leu Gln Lys Arg
Gly Ile Val Glu Gln Cys Cys Thr Ser Ile Cys Ser Leu Tyr Gln Leu Glu Asn Tyr Cys Asn
(SEQ ID NO: 7), beispielsweise die Sequenz des His (B0)-Präproinsulins, oder die Sequenz
Met Ala Thr Thr Ser Thr Gly Asn Ser Ala Arg
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys Ser Leu Tyr Gln Leu Glu Asn Tyr Cys Asn
(SEQ ID NO: 8), beispielsweise die Sequenz des His(B-1), Ala(B0)-Präproinsulins, oder die Sequenz
Met Ala Thr Thr Ser Thr Gly Asn Ser Ala Arg
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly lle Val Glu Gln Cys Cys Thr Ser lle Cys
Ser Leu Tyr Gln Leu Glu Asn Tyr Cys Asn
(SEQ ID NO: 9), beispielsweise die Sequenz des His(B-2), Ala(B-1), Ala(B0)-Präproinsulins.

Die vorliegende Erfindung betrifft auch die vorstehend genannten Vorläufer der Insulinanaloga gemäß der vorliegenden Erfindung, insbesondere die Präproinsuline, die DNA-Sequenzen, welche für einen Vorläufer des Insulinanalogons gemäß der vorliegenden Erfindung kodieren, die Expressionsvehikel, welche eine DNA-Sequenz enthalten, die für einen erfindungsgemäßen Vorläufer des Insulinanalogons gemäß der vorliegenden Erfindung kodiert, sowie eine isolierte Wirtszelle, die mit einem solchen Expressionsvehikel transformiert ist.

Die vorliegende Erfindung betrifft ferner eine pharmazeutische Zubereitung, enthaltend mindestens ein Insulinanalogon und/oder mindestens ein physiologisch verträgliches Salz gemäß der vorliegenden Erfindung.

Vorzugsweise zeichnet sich die pharmazeutische Zubereitung dadurch aus, daß sie das erfindungsgemäße Insulinanalogon und/oder das physiologisch verträgliche Salz desselben in gelöster, amorpher und/oder kristalliner Form enthält.

Die pharmazeutische Zubereitung enthält wahlweise ferner einen Depothilfsstoff, vorzugsweise Protaminsulfat, wobei das Insulinanalogon und/oder das physiologisch verträgliche Salz desselben vorzugsweise mit dem Protaminsulfat in einem Cokristallisat vorliegt.

Die pharmazeutische Zubereitung gemäß der vorliegenden Erfindung kann wahlweise zusätzlich unmodifiziertes Humaninsulin und/oder ein weiteres Insulinanalogon enthalten, vorzugsweise Gly(A21)-Arg(B31)-Arg(B32)-Humaninsulin.

Die vorliegende Erfindung betrifft ferner eine injizierbare Lösung mit Insulinaktivität, welche die pharmazeutische Zubereitung gemäß der vorliegenden Erfindung in gelöster Form enthält, vorzugsweise gekennzeichnet durch einen Gehalt von 1 µg bis 2 mg Zink pro ml, besonders bevorzugt durch einen Gehalt von 5 µg bis 200 µg Zink pro ml.

Die vorliegende Erfindung betrifft ferner die Verwendung des Insulinanalogons und/oder dessen physiologisch verträglichen Salzes gemäß der vorliegenden Erfindung zur Herstellung einer phamazeutischen Zubereitung, welche eine Insulinaktivität mit verzögertem Wirkungseintritt aufweist.

Die eingangs gestellte Aufgabe wird ferner gelöst durch einen Insulin-Zink-Komplex, enthaltend ein Insulinhexamer und 4 bis 10 Zinkionen pro Insulinhexamer, dadurch gekennzeichnet, daß das Insulinhexamer aus sechs Molekülen eines Insulinanalogons der Formel I wie oben beschrieben besteht.

Vorzugsweise erithält der Insulin-Zink-Komplex 5 bis 8 Zinkionen pro Insulinhexamer.

Der Insulin-Zink-Komplex gemäß der vorliegenden Erfindung ist vorzugsweise auch dadurch gekennzeichnet, daß die B-Kette des lnsuünanalogons der Formel I die Sequenz
Phe Val His Gln His Leu Cys Gly Ser His Leu Val Glu Ala Leu Tyr Leu Val Cys Gly Glu Arg Gly Phe Phe Tyr Thr Pro Lys Thr
(SEQ ID NO: 10) aufweist, beispielsweise das His(B3)-Humaninsulin, oder dadurch gekennzeichnet, daß die B-Kette des Insulinanalogons der Formel I die Sequenz
Phe Val Asp Gln-His Leu Cys Gly Ser His Leu Val Glu Ala Leu Tyr Leu Val Cys Gly Glu Arg Gly Phe Phe Tyr Thr Pro Lys Thr
(SEQ ID NO: 11) aufweist, beispielsweise das Asp(B3)-Humaninsulin.

Die vorliegende Erfindung betrifft auch eine pharmazeutische Zubereitung, enthaltend mindestens einen erfindungsgemäßen Insulin-Zink-Komplex sowie eine pharmazeutische Zubereitung, enthaltend den erfindungsgemäßen Insulin-Zink-Komplex um eine saure Lösung.

Die pharmazeutische Zubereitung ist vorzugsweise dadurch gekennzeichnet, daß sie den Insulin-Zink-Komplex in gelöster, amorpher und/oder kristalliner Form enthält.

Die vorliegende Erfindung betrifft auch eine injizierbare Lösung mit Insulinaktivität, enthaltend die pharmazeutische Zubereitung in gelöster Form und ist vorzugsweise gekennzeichnet durch einen Gehalt von 1 µg bis 2 mg Zink pro ml, besonders bevorzugt durch einen Gehalt von 5 µg bis 200 µg Zink pro ml.

Die vorliegende Erfindung betrifft auch die Verwendung des Insulin-Zink-Komplexes zur Herstellung einer phamazeutischen Zubereitung, welche eine Insulinaktivität mit verzögertem Wirkungseintritt aufweist.

Die Insulinanaloga gemäß der vorliegenden Erfindung sind biologisch aktiv und zeigen nach subcutaner Applikation als schwach saure; klare Lösung mit 80 µg Zn⁺⁺/ml (Zink/ml) am Hund eine stark verzögerte Wirkung. Bei dem Insulinanalogon, welches am N-Terminus der B-Kette mit Histidin verlängert ist, das His(BO), des (B30)-Humaninsulin (s. SEQ ID NO.: 3), hängt das Wirkprofil beispielsweise sehr stark von der Menge an zugesetzten Zinkionen ab. Eine zinkfreie Zubereitung besitzt keinerlei Depoteffekt (Gesamtwirkung 6 - 8 h, Beispiel 8) und unterscheidet sich in der Pharmakodynamik kaum von Humaninsulin, während man nach Zusatz von Zinkionen (80 µg/ml) eine starke Wirkungsverzögerung findet (Gesamtwirkung etwa 16 h, Beispiel 8). Der beobachtete Depoteffekt, ist also wesentlich ausgeprägter als der von NPH-Insulin. Darüber hinaus besitzt dieses Analogon den Vorteil, daß die Pharmakodynamik durch Vorgabe des Zinkgehaltes in einer Breite steuerbar ist, wie es mit Humaninsulin nicht möglich ist. Formulierungen mit raschem Wirkungseintritt lassen sich ebenso wie solche mit mäßig oder stark verzögerter Wirkung mit einer Wirksubstanz allein durch die Variation des Zinkgehaltes herstellen. Damit kann das Wirkprofil individuell den Bedürfnissen des Patienten angepaßt werden, entweder unter Verwendung einer Zubereitung mit entsprechend voreingestelltem Zinkgehalt oder durch Mischung von Zubereitungen mit hohem und niedrigem Zinkgehalt durch den Arzt oder den Patienten selbst.

Die hier beschriebenen Analoga sind ferner dadurch charakterisiert, daß sie im Vergleich zu Humaninsulin eine erhöhte Affinität zu Zinkionen aufweisen.

Humaninsulin bildet in wäßriger neutraler Lösung Hexamere, welche jeweils zwei Zinkionen über die His(B10)-Seitenketten komplexieren. Diese Zinkionen lassen sich durch Dialyse gegen wäßrige Puffer im Neutralen nicht entfemen. Unter den gleichen Bedingungen binden die hier beschriebenen Analoga mehr als 4 Zinkionen. Im Falle des erfindungsgemäßen His(B0)-Des(B30)- und His(B3)-Insulin sind dies etwa 7 Zinkionen/Hexamer, bei Asp(B3)-Insulin wurden 4,2 Zinkionen/Hexamer gemessen (Beispiel 9).

Es ist bekannt, daß in neutralen Lösungen Zink zur Bildung höhermolekularer Assoziate und zur Ausfällung des Insulins führt. Nach der Injektion einer schwach sauren zinkhaltigen Zubereitung, die Insulin klar gelöst enthält, kommt es daher im subcutanen Gewebe durch Neutralisation zur Bildung von Insulin-Zinkkomplexen und in der Folge zur Präzipitation des Insulins. Aus diesem Depot geht Insulin wieder in Lösung und gelangt dann verzögert in den Blutstrom und an den Wirkort. Diese Wirkungsverzögerung ist bei Humaninsulin nur schwach, bei den hier beschriebenen Analoga aufgrund der erhöhten Affinität zu Zink jedoch stark ausgebildet. Die erhöhte Zinkbindung stellt daher die Grundlage für die oben beschriebenen zinkabhängige Wirkungsverlängerung dar.
Die vorliegende Erfindung betrifft daher nicht nur die beschriebenen Insulinanaloga sondern auch auf die zugehörigen Insulin-Zink-Komplexe. Diese Komplexe unterscheiden sich von den entsprechenden Humaninsulin-Zink-Komplexen dadurch, daß sie einen höheren Anteil an fest gebundenem Zink aufweisen. Es ist daher naheliegend, daß neben Zink auch andere Übergangsmetallionen wie zum Beispiel Cobalt oder Kupfer zur Bildung entsprechender Komplexe eingesetzt werden können.

### Beispiel 1: Konstruktion des Plasmides plNT345d kodierend für die Variante His(B3)-Präproinsulin.

Das US Patent mit der Patentnummer 5358857 beschreibt das Plasmid pINT90d. DNA dieses Plasmides dient als Ausgangsmaterial zur Konstruktion des Plasmides pINT345d, welches gegenüber plNT90d durch zwei neue Eigenschaften charakterisiert ist. Es kodiert zum einen für ein Präproinsulinanalogon, das in Position 3 der B-Kette die Aminosäure Histidin statt Asparagin enthält und trägt zum anderen unmittelbar vor Beginn der für diese Präproinsulinvariante kodierenden Sequenz eine Erkennungssequenz für das Restriktionsenzym BssH2, so daß die für die N-terminalen 10 Aminosäuren des Präproinsulinanalogons kodierende Sequenz leicht manipulierbar wird, wenn man die Dra3-Spaltstelle im Verlauf der Präproinsulinsequenz berücksichtigt.
Zur Konstruktion des Plasmides pINT345d wird DNA des Plasmides plNT90d in einem Doppelverdauansatz in Position 284bp durch das Restriktionsenzym Ncol und in Position 351 bp durch das Restriktionsenzym Dra3 gespalten, so daß zwei Fragmente enstehen. Nach gelelektrophoretischer Auftrennung des Spaltgemisches wird das große Restplasmid-DNA-Fragment isoliert.
Dieses DNA-Fragment wird dann mit dem synthetischen DNA-Fragment der Form in einer T4-DNA-Ligasereaktion umgesetzt. Kompetente E.coli K12-Zellen werden mit dem Ligationsgemisch transformiert und der Transformationsansatz auf NA-Platten, die 20mg/l Ampicillin enthalten, ausplattiert. Die Platten werden über Nacht bei 37° C inkubiert. Von entstandenen Kolonien wird Plasmid-DNA isoliert mit dem Restriktionsenzym BssH2 gespalten. Die gewünschte Plasmid-DNA wird dabei linearisiert und unterscheidet sich so von pINT90d-DNA, die keine BssH2-Schnittstelle enthält und entsprechend nicht gespalten wird.
Die Plasmid-DNA eines Klones, die sich richtig verhält, wird mit pINT345d bezeichnet.
Sie dient als Ausgangsmaterial für die die Konstruktion der im folgenden beschriebenen Präproinsulinvarianten.

### Beispiel 2: Konstruktion des Plasmides pINT342d kodierend für die Variante His(B0)-Präproinsulin

DNA des Plasmides pINT345d wird mit den Enzymen BssH2 und Dra3 doppelverdaut und das große Restplasmidfragement nach gelektrophoretischer Trennung isoliert. Dieses DNA-Fragement wird mit dem synthetischen DNA-Fragment der Form in einer T4-DNA-Ligaseraktion umgesetzt. Es entsteht das Plasmid pINT342d, das durch eine zusätzliche Hpa1-Schnittstelle gegenüber dem Ausgangsplasmid charakterisiert ist. Das Plasmid kodiert für eine Präproinsulinvariante die in Position B0 ein Histidin aufweist.

### Beispiel 3: Konstruktion des Plasmides plNT343d kodierend für die Variante His(B-1), Ala(B0)-Präproinsulin

DNA des in Beispiel b beschriebenen Restplasmidfragmentes wird mit einem synthetischen DNA-Fragment der Form in einer T4-DNA-Ligasereaktion umgesetzt. Es entsteht das Plasmid pINT343d, das wie auch pINT342d gegenüber dem Ausgangsvektor eine zusätzliche Hpa1-Schnittstelle enthält.

### Beispiel 4: Konstruktion des Plasmides pINT344d kodierend für die Variante His(B-2),Ala(B-1),Ala(BO)-Präproinsulin

DNA des in Beispiel b beschriebenen Restplasmidfragmentes wird mit einem synthetischen DNA-Fragment der Form in einer T4-DNA-Ligasereaktion umgesetzt. Es entsteht das Plasmid pINT344d, das gegenüber dem Ausgangsvektor durch eine zusätzliche Hpa1-Schnittstelle charakterisiert ist.

### Beispiel 5: Expression der konstruierten Insulinvarianten

Die Plasmide pINT 342d, 343d und 344d werden beispielhaft jeweils nach E.coli K12 W3110 transformiert. Rekombinante Bakterien, welche die Plasmide für die jeweilige Variante enthalten, werden dann gemäß Beispiel 4 des US Patentes mit der Patentnummer 5227293 fermentiert und so der gewünschte Rohstoff zur Erzeugung der jeweiligen Insulinvariante erzeugt.

### Beispiel 6: Herstellung von His(B0),Des(B30)-Insulin

Gemäß Beispiel 5 wird die Präproinsulinvariante in E.coli exprimiert und in Form von Einschlußkörperchen (inclusion bodies) nach Zellaufschluß durch Zentrifugation isoliert. Die Einschlußkörperchen werden in Harnstoff (8 mol/l) gelöst, einer Sulfitolyse unterzogen und durch Anionenaustauscher (Q-Sepharose) und Gelpermeationschromatographie (Sephacryl S 200) gereinigt. Die bei der Chromatographie eingesetzten Puffer enthalten 4 M Harnstoff und 50 mM Tris/HCI (Tris(hydroxymethyl)aminomethan/HCI) pH 8,5. Die fraktionierte Elution am Anionenaustauscher erfolgt durch Anlegen eines Gradienten von 0 bis 0,5 M NaCl. Die Konzentration des Harnstoffs wird anschließend durch Ultrafiltration und Verdünnen auf < 1 M reduziert und das Präproinsulin-S-Sulfonat durch Fällung bei pH 4 isoliert und zuletzt getrocknet.
Zur Ausbildung der korrekten Disulfidbrücken, wie sie in natürlichem Proinsulin vorliegen, wird das Präproinsulin-S-Sulfonat bei einer Konzentration von 0,3 g/l in einem Puffer, der 20 mM Glycin enthält, bei pH 10,8 gelöst, mit Merkaptoethanol versetzt (etwa 25-50 mol/mol Präproinsulin) und über Nacht bei 4 °C gerührt. Der Ansatz wird anschließend mit Phosphorsäure auf pH 3,5 gestellt und zentrifugiert. Das im Überstand enthaltene Präproinsulin wird zur Überführung in Insulin nach Zugabe von Tris (25 mM) auf pH 8,2 gestellt und mit Trypsin (1,5 mg/g Präproinsulin) versetzt. Der Verlauf der proteolytischen Spaltung wird mittels Reversed Phase HPLC verfolgt. Nach etwa 6 Stunden enthält der Ansatz einen hohen Anteil an His(BO),Des(B30)-Insulin. Die Reaktion wird durch Ansäuern auf pH 3,5 beendet. Die Reinigung des Insulinanalogons erfolgt durch lonenaustauscher-Chromatographie (S-Hyper-D, Sepracor) und Reversed Phase Chromatographie (PLRP-S RP300, Polymer Laboratories). Die Ionenaustauscherchromatographie wird in einem Puffer durchgeführt, der 30 % 2-Propanol und 50 mM Milchsäure (pH 3,5) enthält. Die Elution des gebundenen Insulins erfolgt durch einen linearen Gradienten von 0 bis 0,5 M NaCl. Die Reversed Phase Chromatographie erfolgt in 0,1 % Trifluoressigsäure, der zur Elution steigende Mengen an Acetonitril zugemischt werden. Das Produkt wird durch Ausfällung bei pH 5,4 isoliert und lyophilisiert.

### Beispiel 7: Formulierung von Insulinanaloga zur parenteralen Applikation

Die Zubereitungen enthalten je ml 40 bzw. 100 IE Insulin (1 IE entspricht etwa 6,2 nmol), 20 mg 85 % Glycerin, 2,7 mg m-Kresol und gegebenenfalls Zink⁺⁺ (als Zinkchlorid) in wässriger, steriler Lösung bei pH 4.

### Beispiel 8: Wirkprofil von His(B0),Des(B30)-Insulin am Hund

Jeweils 6 Hunde (Beagles) erhielten subcutane Applikationen einer Zubereitung mit 40 U/ml (Beispiel 7) und dem angegebenen Gehalt an Zink. Die Dosis betrug 0,3 IE/kg. Im weiteren Verlauf des Versuchs wurde nach den angegebenen Zeiten die Konzentration der Blutglucose gemessen. Die Werte wurden prozentual auf den jeweiligen Ausgangswert normiert und gemittelt.

| *Zeit (Stunden)* | *0* | *1* | *2* | *3* | *4* | *5* | *6* | *7* | *8* | *9* | *10* | *12* |
|---|---|---|---|---|---|---|---|---|---|---|---|---|
| Zinkfrei | 100 | 59 | 50 | 61 | 75 | 84 | 89 | 98 | 103 | 97 | 104 | 100 |
| 80 µg | 100 | 97 | 83 | 75 | 65 | 56 | 51 | 58 | 68 | 72 | 78 | 82 |
| Zink⁺⁺/ml | | | | | | | | | | | | |

### Beispiel 9: Zinkbindung von Insulinanaloga

Eine Zubereitung von Insulin (0,3 mM Insulin, 0,13 M NaCl, 0,1 % Phenol, 100 µg/ml Zink⁺⁺ (als Zinkchlorid), 25 mM Tris/HCl, pH 7,4) wurde extensiv gegen zinkfreien neutralen Puffer dialysiert (3h gegen 0,15 M NaCl, 10 mM Tris/HCl pH 7,4 bei Raumtemperatur, 72 Stunden gegen 10 mM Tris/HCl pH 7,4 bei 15 °C und nochmals 16 h gegen 10 mM Tris/HCl pH 7.4 bei 15 °C). Danach wurden die Dialysate angesäuert und analysiert. Die Konzentration von Insulin wurde durch Reversed Phase-HPLC und die des Zink durch Atomabsorptionsspektroskopie bestimmt. Die Zinkwerte wurden mit dem Zinkgehalt eines Kontrollansatzes, der kein Insulin enthielt, korrigiert.

### Zinkbindung

| *Insulin* | *mol Zinklmol Hexamer* |
|---|---|
| Humaninsulin | 2,5 |
| His(B3)-Insulin | 6,9 |
| Asp(B3)-Insulin | 4,2 |
| His(B0),Des(B30)-Insulin | 6,8 |

## Patentansprüche

1. Insulinanalogon oder ein physiologisch verträgliches Salz desselben der Formel I 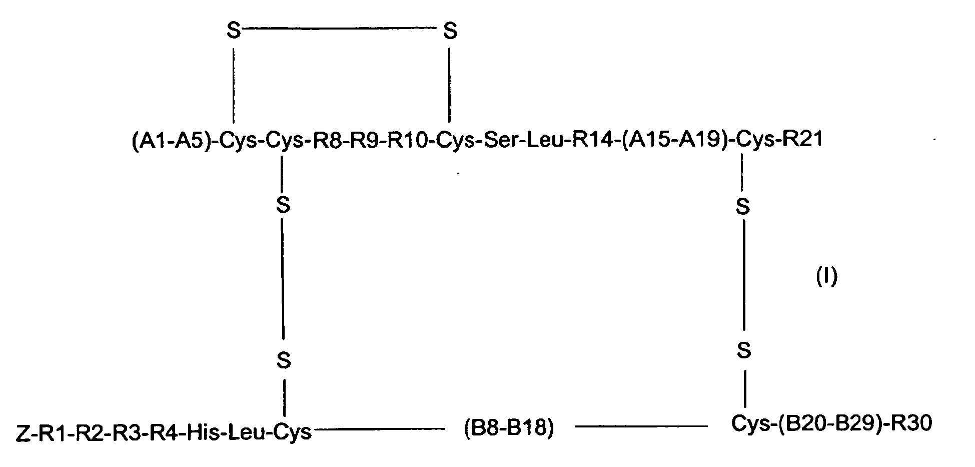 worin bedeuten
| | |
|---|---|
| (A1-A5) | die Aminosäurereste in den Positionen A1 bis A5 der A-Kette von Humaninsulin oder tierischem Insulin, |
| (A15-A19) | die Aminosäurereste in den Positionen A15 bis A19 der A-Kette von Humaninsulin oder tierischem Insulin, |
| (B8-B18) | die Aminosäurereste in den Positionen B8 bis B18 der B-Kette von Humaninsulin oder tierischem Insulin, |
| (B20-B29) | die Aminosäurereste in den Positionen B20 bis B29 der B-Kette von Humaninsulin oder tierischem Insulin, |
| R8 | Thr oder Ala, |
| R9 | Ser oder Gly, |
| R10 | Ile oder Val, |
| R14 | Tyr, His, Asp oder Glu, |
| R21 | Asn, Asp, Gly, Ser, Thr, Ala, Glu oder Gln, |
| R1 | ein beliebiger genetisch kodierbarer Aminosäurerest, abwesend oder ein Wasserstoffatom, |
| R2 | Val, Ala oder Gly, |
| R3 | Asn, His, Glu oder Asp, |
| R4 | Ala, Ser, Thr, Asn, Asp, Gln, Gly oder Glu, |
| R30 | ein beliebiger genetisch kodierbarer Aminosäurerest oder -OH, |
| Z | ein Peptidrest mit 1 bis 4 genetisch kodierbaren Aminosäureresten, enthaltend 1 bis 4 Histidinreste (His), |
mit der Maßgabe, daß sich das Insulinanalogon oder das physiologisch verträgliche Salz desselben der Formel I nicht allein durch Variation der Aminosäurereste in den Positionen R3 oder R3 in Verbindung mit R21 oder R3 in Verbindung mit R4 in Formel I von Humaninsulin unterscheidet.

2. Insulinanalogon oder ein physiologisch verträgliches Salz desselben nach Anspruch 1, **dadurch gekennzeichnet, daß** R8 Thr, R9 Ser und R10 Ile bedeuten.

3. Insulinanalogon oder ein physiologisch verträgliches Salz desselben nach Anspruch 1 oder 2, **dadurch gekennzeichnet, daß** R1 Phe, His, Asn, Asp oder Gly bedeutet.

4. Insulinanalogon oder ein physiologisch verträgliches Salz desselben nach einem oder mehreren der Ansprüche 1 bis 3, **dadurch gekennzeichnet, daß** R30 Thr, Ala oder Ser bedeutet.

5. Insulinanalogon oder ein physiologisch verträgliches Salz desselben nach einem oder mehreren der Ansprüche 1 bis 4, **dadurch gekennzeichnet, daß** R21 Asn und R1 Phe bedeuten.

6. Insulinanalogon oder ein physiologisch verträgliches Salz desselben nach einem oder mehreren der Ansprüche 1 bis 5, **dadurch gekennzeichnet, daß** R2 Val, R3 Asn und R4 Gln bedeuten.

7. Insulinanalogon oder ein physiologisch verträgliches Salz desselben nach einem oder mehreren der Ansprüche 1 bis 6, **dadurch gekennzeichnet, daß** R14 Tyr bedeutet.

8. Insulinanalogon oder ein physiologisch verträgliches Salz desselben nach einem oder mehreren der Ansprüche 1 bis 6, **dadurch gekennzeichnet, daß** R14 His bedeutet.

9. Insulinanalogon oder ein physiologisch verträgliches Salz desselben nach einem oder mehreren der Ansprüche 1 bis 6, **dadurch gekennzeichnet, daß** R14 Asp bedeutet.

10. Insulinanalogon oder ein physiologisch verträgliches Salz desselben nach einem oder mehreren der Ansprüche 1 bis 6, **dadurch gekennzeichnet, daß** R14 Glu bedeutet.

11. Insulinanalogon oder ein physiologisch verträgliches Salz desselben nach einem oder mehreren der Ansprüche 1 bis 10, **dadurch gekennzeichnet, daß** R30 Thr bedeutet.

12. Insulinanalogon oder ein physiologisch verträgliches Salz desselben nach einem oder mehreren der Ansprüche 1 bis 10, **dadurch gekennzeichnet, daß** R30 Ala bedeutet.

13. Insulinanalogon oder ein physiologisch verträgliches Salz desselben nach einem oder mehreren der Ansprüche 1 bis 10, **dadurch gekennzeichnet, daß** R30 Ser bedeutet.

14. Insulinanalogon oder ein physiologisch verträgliches Salz desselben nach einem oder mehreren der Ansprüche 1 bis 3 und 5 bis 10, **dadurch gekennzeichnet, daß** R30 -OH bedeutet.

15. Insulinanalogon oder ein physiologisch verträgliches Salz desselben nach einem oder mehreren der Ansprüche 1 bis 14, **dadurch gekennzeichnet, daß** Z His bedeutet.

16. Insulinanalogon oder ein physiologisch verträgliches Salz desselben nach einem oder mehreren der Ansprüche 1 bis 14, **dadurch gekennzeichnet, daß** Z His-Ala- bedeutet.

17. Insulinanalogon oder ein physiologisch verträgliches Salz desselben nach einem oder mehreren der Ansprüche 1 bis 14, **dadurch gekennzeichnet, daß** Z His-Ala-Ala- bedeutet.

18. Insulinanalogon oder ein physiologisch verträgliches Salz desselben nach Anspruch 15, **dadurch gekennzeichnet, daß** die B-Kette die Sequenz
His Phe Val Asn Gln His Leu Cys Gly Ser His Leu Val Glu Ala Leu Tyr Leu Val Cys Gly Glu Arg Gly Phe Phe Tyr Thr Pro Lys
aufweist (SEQ ID NO: 3).

19. Insulinanalogon oder ein physiologisch verträgliches Salz desselben nach Anspruch 15, **dadurch gekennzeichnet, daß** die B-Kette die Sequenz
His Phe Val Asn Gln His Leu Cys Gly Ser His Leu Val Glu Ala Leu Tyr Leu Val Cys Gly Glu Arg Gly Phe Phe Tyr Thr Pro Lys Thr
aufweist (SEQ ID NO: 4).

20. Insulinanalogon oder ein physiologisch verträgliches Salz desselben nach Anspruch 16, **dadurch gekennzeichnet, daß** die B-Kette die Sequenz
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
aufweist (SEQ ID NO: 5).

21. Insulinanalogon oder ein physiologisch verträgliches Salz desselben nach Anspruch 17, **dadurch gekennzeichnet, daß** die B-Kette die Sequenz
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
aufweist (SEQ ID NO: 6).

22. Verfahren zur Herstellung eines Insulinanalogons oder eines physiologisch verträglichen Salzes desselben gemäß einem oder mehreren der Ansprüche 1 bis 21, umfassend die Konstruktion eines replizierbaren Expressionsvehikels, welches eine DNA-Sequenz enthält, die für einen Vorläufer des Insulinanalogons mit der allgemeinen Aminosäuresequenz II kodiert
Met-X²ₘ-(Arg)ₚ-Z-R1-R2-R3-R4-His-Leu-Cys-(B8-B18)-Cys-(B20-B29)-R30-X¹ₙ-Arg-(A1-A5)-Cys-Cys-R8-Ser-R10-Cys-Ser-Leu-R14-(A15-A19)-Cys-R21 II,
worin
| | |
|---|---|
| X¹ₙ | eine Peptidkette mit n Aminosäurresten ist, wobei n eine ganze Zahl von 0 bis 34 bedeutet, |
| X²ₘ | eine Peptidkette mit m Aminosäurresten ist, wobei m eine ganze Zahl von 0 bis 20 bedeutet, |
| p | 0, 1 oder 2, |
| R30 | ein beliebiger genetisch kodierbarer Aminosäurerest bedeutet oder abwesend ist und |
| Z | ein Peptidrest mit 1 bis 4 genetisch kodierbaren Aminosäureresten, enthaltend 1 bis 4 Histidinreste (His) bedeutet |
und die übrigen Variablen die in Anspruch 1 genannten Bedeutungen haben, wobei auch die in Anspruch 1 genannten Maßgaben gelten, Expression in einer Wirtszelle und Freisetzung des Insulinanalogons aus dessen Vorläufer mit chemischen und/oder enzymatischen Methoden.

23. Verfahren nach Anspruch 22, **dadurch gekennzeichnet, daß** die Wirtszelle ein Bakterium ist.

24. Verfahren nach Anspruch 23, **dadurch gekennzeichnet, daß** das Bakterium E. coli ist.

25. Verfahren nach Anspruch 22, **dadurch gekennzeichnet, daß** die Wirtszelle eine Hefe ist.

26. Verfahren nach Anspruch 25, **dadurch gekennzeichnet, daß** die Hefe Saccharomyces cerevisiae ist.

27. Verfahren nach einem der Ansprüche 22 bis 26 zur Herstellung eines Insulinanalogons gemäß Anspruch 19, **dadurch gekennzeichnet, daß** der Vorläufer des Insulinanalogons die Sequenz
Met Ala Thr Thr Ser Thr Gly Asn Ser Ala Arg
His Phe Val Asn Gin His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gin Val Gly Gln Val Glu Leu Gly Gly Gly Pro Gly Ala Gly Ser Leu Gln Pro Leu Ala Leu Glu Gly Ser Leu Gin Lys Arg
Gly Ile Val Glu Gin Cys Cys Thr Ser Ile Cys Ser Leu Tyr Gin Leu Glu Asn Tyr Cys Asn
aufweist (SEQ ID NO: 7).

28. Verfahren nach einem der Ansprüche 22 bis 26 zur Herstellung eines Insulinanalogons gemäß Anspruch 20, **dadurch gekennzeichnet, daß** der Vorläufer des Insulinanalogons die Sequenz
Met Ala Thr Thr Ser Thr Gly Asn Ser Ala Arg
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gin Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gin Cys Cys Thr Ser Ile Cys Ser Leu Tyr Gin Leu Glu Asn Tyr Cys Asn
aufweist (SEQ ID NO: 8).

29. Verfahren nach einem der Ansprüche 22 bis 26 zur Herstellung eines Insulinanalogons gemäß Anspruch 21, **dadurch gekennzeichnet, daß** der Vorläufer des Insulinanalogons die Sequenz
Met Ala Thr Thr Ser Thr Gly Asn Ser Ala Arg
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys
Ser Leu Tyr Gln Leu Glu Asn Tyr Cys Asn
aufweist (SEQ ID NO: 9).

30. Vorläufer des Insulinanalogons gemäß Anspruch 22.

31. Vorläufer des Insulinanalogons gemäß Anspruch 27.

32. Vorläufer des Insulinanalogons gemäß Anspruch 28.

33. Vorläufer des Insulinanalogons gemäß Anspruch 29.

34. DNA-Sequenz, welche für einen Vorläufer des Insulinanalogons gemäß Anspruch 30 kodiert.

35. DNA-Sequenz, welche für einen Vorläufer des Insulinanalogons gemäß Anspruch 31 kodiert.

36. DNA-Sequenz, welche für einen Vorläufer des Insulinanalogons gemäß Anspruch 32 kodiert.

37. DNA-Sequenz, welche für einen Vorläufer des Insulinanalogons gemäß Anspruch 33 kodiert.

38. Expressionsvehikel, enthaltend eine DNA-Sequenz gemäß Anspruch 34.

39. Expressionsvehikel, enthaltend eine DNA-Sequenz gemäß Anspruch 35.

40. Expressionsvehikel, enthaltend eine DNA-Sequenz gemäß Anspruch 36.

41. Expressionsvehikel, enthaltend eine DNA-Sequenz gemäß Anspruch 37.

42. Isolierte Wirtszelle, die mit einem Expressionsvehikel gemäß einem der Ansprüche 38 bis 41 transformiert ist.

43. Pharmazeutische Zubereitung, enthaltend mindestens ein Insulinanalogon und/oder mindestens ein physiologisch verträgliches Salz desselben gemäß einem oder mehreren der Ansprüche 1 bis 21.

44. Pharmazeutische Zubereitung nach Anspruch 43, **dadurch gekennzeichnet, daß** sie das Insulinanalogon und/oder das physiologisch verträgliche Salz desselben in gelöster, amorpher und/oder kristalliner Form enthält.

45. Pharmazeutische Zubereitung nach Ansprüche 43 oder 44, **dadurch gekennzeichnet, daß** sie ferner einen Depothilfsstoff enthält.

46. Pharmazeutische Zubereitung nach Anspruch 45, **dadurch gekennzeichnet, daß** der Depothilfsstoff Protaminsulfat ist, wobei das Insulinanalogon und/oder das physiologisch verträgliche Salz desselben mit dem Protaminsulfat in einem Cokristallisat vorliegt.

47. Pharmazeutische Zubereitung nach einem oder mehreren der Ansprüche 43 bis 46, **gekennzeichnet durch** einen zusätzlichen Gehalt an unmodifiziertem Humaninsulin.

48. Pharmazeutische Zubereitung nach einem oder mehreren der Ansprüche 43 bis 47, **gekennzeichnet durch** einen zusätzlichen Gehalt an einem weiteren Insulinanalogon.

49. Pharmazeutische Zubereitung nach Anspruch 48, **dadurch gekennzeichnet, daß** das weitere Insulinanalogon Gly(A21)-Arg(B31)-Arg(B32)-Humaninsulin ist.

50. Injizierbare Lösung mit Insulinaktivität, enthaltend die pharmazeutische Zubereitung gemäß einem oder mehreren der Ansprüche 43 bis 49 in gelöster Form.

51. Injizierbare Lösung nach Anspruch 50, **gekennzeichnet durch** einen Gehalt von 1 µg bis 2 mg Zink pro ml.

52. Injizierbare Lösung nach Anspruch 51, **gekennzeichnet durch** einen Gehalt von 5 µg bis 200 µg Zink pro ml.

53. Verwendung des Insulinanalogons und/oder dessen physiologisch verträglichen Salzes gemäß einem oder mehreren der Ansprüche 1 bis 21 zur Herstellung einer phamazeutischen Zubereitung, welche eine Insulinaktivität mit verzögertem Wirkungseintritt aufweist.

54. Insulin-Zink-Komplex, enthaltend ein Insulinhexamer und 4 bis 10 Zinkionen pro Insulinhexamer, **dadurch gekennzeichnet, daß** das Insulinhexamer aus sechs Molekülen eines Insulinanalogons der Formel I gemäß einem oder mehreren der Ansprüche 1 bis 21 besteht.

55. Insulin-Zink-Komplex nach Anspruch 54, enthaltend 5 bis 8 Zinkionen pro Insulinhexamer.

56. Insulin-Zink-Komplex nach Anspruch 54 oder 55, **dadurch gekennzeichnet, daß** die B-Kette des Insulinanalogons der Formel I die Sequenz
Phe Val His Gin His Leu Cys Gly Ser His Leu Val Glu Ala Leu Tyr Leu Val Cys Gly Glu Arg Gly Phe Phe Tyr Thr Pro Lys Thr
(SEQ ID NO: 10) aufweist.

57. Insulin-Zink-Komplex nach Anspruch 54 oder 55, **dadurch gekennzeichnet, daß** die B-Kette des Insulinanalogons der Formel I die Sequenz
Phe Val Asp Gln His Leu Cys Gly Ser His Leu Val Glu Ala Leu Tyr Leu Val Cys Gly Glu Arg Gly Phe Phe Tyr Thr Pro Lys Thr
(SEQ ID NO: 11) aufweist.

58. Pharmazeutische Zubereitung, enthaltend mindestens einen Insulin-Zink-Komplex gemäß einem oder mehreren der Ansprüche 54 bis 57.

59. Pharmazeutische Zubereitung gemäß Anspruch 58, enthaltend eine saure Lösung.

60. Pharmazeutische Zubereitung nach Anspruch 59, enthaltend eine saure Lösung des Insulinanalogons und/oder des physiologisch verträglichen Salzes desselben gemäß einem oder mehreren der Ansprüche 1 bis 21 mit einer entsprechende Menge an Zinkionen, welche die Ausbildung eines Insulin-Zink-Komplexes gemäß Anspruch 54 ermöglicht.

61. Pharmazeutische Zubereitung nach Anspruch 59, enthaltend einen Insulin-Zink-Komplex gemäß einem der Ansprüche 56 oder 57.

62. Pharmazeutische Zubereitung nach einem oder mehreren der Ansprüche 58 bis 61, **dadurch gekennzeichnet, daß** sie den Insulin-Zink-Komplex in gelöster, amorpher und/oder kristalliner Form enthält.

63. Injizierbare Lösung mit Insulinaktivität, enthaltend die pharmazeutische Zubereitung gemäß Anspruch 62 in gelöster Form.

64. Injizierbare Lösung nach Anspruch 63, **gekennzeichnet durch** einen Gehalt von 1 µg bis 2 mg Zink pro ml.

65. Injizierbare Lösung nach Anspruch 64, **gekennzeichnet durch** einen Gehalt von 5 µg bis 200 µg Zink pro ml.

66. Verwendung des Insulin-Zink-Komplex gemäß einem oder mehreren der Ansprüche 54 bis 57 zur Herstellung einer phamazeutischen Zubereitung, welche eine Insulinaktivität mit verzögertem Wirkungseintritt aufweist.

## Claims

1. An insulin analog or a physiologically tolerable salt thereof of the formula I 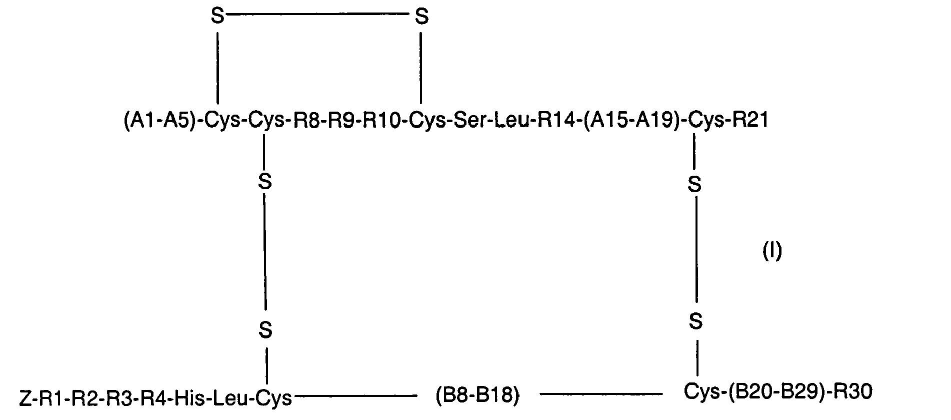 in which
| | |
|---|---|
| (A1-A5) | are the amino acid residues in the positions A1 to A5 of the A chain of human insulin or animal insulin, |
| (A15-A19) | are the amino acid residues in the positions A15 to A19 of the A chain of human insulin or animal insulin, |
| (B8-B18) | are the amino acid residues in the positions B8 to B18 of the B chain of human insulin or animal insulin, |
| (B20-B29) | are the amino acid residues in the positions B20 to B29 of the B chain of human insulin or animal insulin, |
| R8 | is Thr or Ala, |
| R9 | is Ser or Gly, |
| R10 | is Ile or Val, |
| R14 | is Tyr, His, Asp or Glu, |
| R21 | is Asn, Asp, Gly, Ser, Thr, Ala, Glu or Gln, |
| R1 | is any desired genetically encodable amino acid residue, absent or a hydrogen atom, |
| R2 | is Val, Ala or Gly, |
| R3 | is Asn, His, Glu or Asp, |
| R4 | is Ala, Ser, Thr, Asn, Asp, Gln, Gly or Glu, |
| R30 | is any desired genetically encodable amino acid residue or -OH, |
| Z | is a peptide residue having 1 to 4 genetically encodable amino acid residues, comprising 1 to 4 histidine residues (His), |
with the proviso that the insulin analog or the physiologically tolerable salt thereof of the formula I differs from human insulin not only by variation of the amino acid residues in the positions R3 or R3 in combination with R21 or R3 in combination with R4 in formula I.

2. An insulin analog or a physiologically tolerable salt thereof as claimed in claim 1, wherein R8 is Thr, R9 is Ser and R10 is Ile.

3. An insulin analog or a physiologically tolerable salt thereof as claimed in claim 1 or 2, wherein R1 is Phe, His, Asn, Asp or Gly.

4. An insulin analog or a physiologically tolerable salt thereof as claimed in one or more of claims 1 to 3, wherein R30 is Thr, Ala or Ser.

5. An insulin analog or a physiologically tolerable salt thereof as claimed in one or more of claims 1 to 4, wherein R21 is Asn and R1 is Phe.

6. An insulin analog or a physiologically tolerable salt thereof as claimed in one or more of claims 1 to 5, wherein R2 is Val, R3 is Asn and R4 is Gln.

7. An insulin analog or a physiologically tolerable salt thereof as claimed in one or more of claims 1 to 6, wherein R14 is Tyr.

8. An insulin analog or a physiologically tolerable salt thereof as claimed in one or more of claims 1 to 6, wherein R14 is His.

9. An insulin analog or a physiologically tolerable salt thereof as claimed in one or more of claims 1 to 6, wherein R14 is Asp.

10. An insulin analog or a physiologically tolerable salt thereof as claimed in one or more of claims 1 to 6, wherein R14 is Glu.

11. An insulin analog or a physiologically tolerable salt thereof as claimed in one or more of claims 1 to 10, wherein R30 is Thr.

12. An insulin analog or a physiologically tolerable salt thereof as claimed in one or more of claims 1 to 10, wherein R30 is Ala.

13. An insulin analog or a physiologically tolerable salt thereof as claimed in one or more of claims 1 to 10, wherein R30 is Ser.

14. An insulin analog or a physiologically tolerable salt thereof as claimed in one or more of claims 1 to 3 and 5 to 10, wherein R30 is -OH.

15. An insulin analog or a physiologically tolerable salt thereof as claimed in one or more of claims 1 to 14, wherein Z is His.

16. An insulin analog or a physiologically tolerable salt thereof as claimed in one or more of claims 1 to 14, wherein Z is His-Ala-.

17. An insulin analog or a physiologically tolerable salt thereof as claimed in one or more of claims 1 to 14, wherein Z is His-Ala-Ala-.

18. An insulin analog or a physiologically tolerable salt thereof as claimed in claim 15, wherein the B chain has the sequence
His Phe Val Asn Gln His Leu Cys Gly Ser His Leu Val Glu Ala Leu Tyr Leu Val Cys Gly Glu Arg Gly Phe Phe Tyr Thr Pro Lys
(SEQ ID NO: 3).

19. An insulin analog or a physiologically tolerable salt thereof as claimed in claim 15, wherein the B chain has the sequence
His Phe Val Asn Gln His Leu Cys Gly Ser His Leu Val Glu Ala Leu Tyr Leu Val Cys Gly Glu Arg Gly Phe Phe Tyr Thr Pro Lys Thr
(SEQ ID NO: 4).

20. An insulin analog or a physiologically tolerable salt thereof as claimed in claim 16, wherein the B chain has the sequence
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
(SEQ ID NO: 5).

21. An insulin analog or a physiologically tolerable salt thereof as claimed in claim 17, wherein the B chain has the sequence
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
(SEQ ID NO: 6).

22. A process for the preparation of an insulin analog or of a physiologically tolerable salt thereof as claimed in one or more of claims 1 to 21, comprising the construction of a replicable expression vehicle which contains a DNA sequence which codes for a precursor of the insulin analog having the amino acid sequence II
Met-X²ₘ-(Arg)ₚ-Z-R1-R2-R3-R4-His-Leu-Cys-(B8-B18)-Cys-(B20-B29)-R30-X¹ₙ-Arg-(A1-A5)-Cys-Cys-R8-Ser-R10-Cys-Ser-Leu-R14-(A15-A19)-Cys-R21 II,
in which
| | |
|---|---|
| X¹ₙ | is a peptide chain having n amino acid residues, where n is an integer from 0 to 34, |
| X²ₘ | is a peptide chain having m amino acid residues, where m is an integer from 0 to 20, |
| p | is 0, 1 or 2, |
| R30 | is any desired genetically encodable amino acid residue or is absent and |
| Z | is a peptide residue having 1 to 4 genetically encodable amino acid residues, comprising 1 to 4 histidine residues (His) |
and the other variables have the meanings mentioned in claim 1, where the provisos mentioned in claim 1 also apply, expression in a host cell and release of the insulin analog from its precursor using chemical and/or enzymatic methods.

23. A process as claimed in claim 22, wherein the host cell is a bacterium.

24. The process as claimed in claim 23, wherein the bacterium is E. coli.

25. The process as claimed in claim 22, wherein the host cell is a yeast.

26. The process as claimed in claim 25, wherein the yeast is Saccharomyces cerevisiae.

27. The process as claimed in one of claims 22 to 26 for the preparation of an insulin analog as claimed in claim 19, wherein the precursor of the insulin analog has the sequence
Met Ala Thr Thr Ser Thr Gly Asn Ser Ala Arg
His Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gin Cys Cys Thr Ser Ile Cys Ser Leu Tyr Gln Leu Glu Asn Tyr Cys Asn
(SEQ ID NO: 7).

28. The process as claimed in one of claims 22 to 26 for the preparation of an insulin analog as claimed in claim 20, wherein the precursor of the insulin analog has the sequence
Met Ala Thr Thr Ser Thr Gly Asn Ser Ala Arg
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys Ser Leu Tyr Gln Leu Glu Asn Tyr Cys Asn
(SEQ ID NO: 8).

29. The process as claimed in one of claims 22 to 26 for the preparation of an insulin analog as claimed in claim 21, wherein the precursor of the insulin analog has the sequence
Met Ala Thr Thr Ser Thr Gly Asn Ser Ala Arg
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys
Ser Leu Tyr Gln Leu Glu Asn Tyr Cys Asn
(SEQ ID NO: 9).

30. A precursor of the insulin analog as set forth in claim 22.

31. A precursor of the insulin analog as set forth in claim 27.

32. A precursor of the insulin analog as set forth in claim 28.

33. A precursor of the insulin analog as set forth in claim 29.

34. A DNA sequence which codes for a precursor of the insulin analog as claimed in claim 30.

35. A DNA sequence which codes for a precursor of the insulin analog as claimed in claim 31.

36. A DNA sequence which codes for a precursor of the insulin analog as claimed in claim 32.

37. A DNA sequence which codes for a precursor of the insulin analog as claimed in claim 33.

38. An expression vehicle comprising a DNA sequence as claimed in claim 34.

39. An expression vehicle comprising a DNA sequence as claimed in claim 35.

40. An expression vehicle comprising a DNA sequence as claimed in claim 36.

41. An expression vehicle comprising a DNA sequence as claimed in claim 37.

42. An isolated host cell which is transformed using an expression vehicle as claimed in one of claims 38 to 41.

43. A pharmaceutical preparation comprising at least one insulin analog and/or at least one physiologically tolerable salt thereof as claimed in one or more of claims 1 to 21.

44. The pharmaceutical preparation as claimed in claim 43, which contains the insulin analog and/or the physiologically tolerable salt thereof in dissolved, amorphous and/or crystalline form.

45. The pharmaceutical preparation as claimed in claim 43 or 44, which furthermore contains a depot auxiliary.

46. The pharmaceutical preparation as claimed in claim 45, wherein the depot auxiliary is protamine sulfate, the insulin analog and/or the physiologically tolerable salt thereof being present in a cocrystallizate with the protamine sulfate.

47. The pharmaceutical preparation as claimed in one or more of claims 43 to 46, additionally containing unmodified human insulin.

48. The pharmaceutical preparation as claimed in one or more of claims 43 to 47, additionally containing a further insulin analog.

49. The pharmaceutical preparation as claimed in claim 48, wherein the further insulin analog is Gly(A21)-Arg(B31)-Arg(B32)-human insulin.

50. An injectable solution having insulin activity, comprising the pharmaceutical preparation as claimed in one or more of claims 43 to 49 in dissolved form.

51. The injectable solution as claimed in claim 50, containing 1 µg to 2 mg of zinc per ml.

52. The injectable solution as claimed in claim 51, containing 5 µg to 200 µg of zinc per ml.

53. The use of the insulin analog and/or its physiologically tolerable salt as claimed in one or more of claims 1 to 21 for the production of a pharmaceutical preparation which has an insulin activity with a delayed onset of action.

54. An insulin-zinc complex comprising an insulin hexamer and 4 to 10 zinc ions per insulin hexamer, wherein the insulin hexamer consists of six molecules of an insulin analog of the formula I as claimed in one or more of claims 1 to 21.

55. The insulin-zinc complex as claimed in claim 54, comprising 5 to 8 zinc ions per insulin hexamer.

56. The insulin-zinc complex as claimed in claim 54 or 55, wherein the B chain of the insulin analog of the formula I has the sequence
Phe Val His Gln His Leu Cys Gly Ser His Leu Val Glu Ala Leu Tyr Leu Val Cys Gly Glu Arg Gly Phe Phe Tyr Thr Pro Lys Thr
(SEQ ID NO: 10).

57. The insulin-zinc complex as claimed in claim 54 or 55, wherein the B chain of the insulin analog of the formula I has the sequence
Phe Val Asp Gln His Leu Cys Gly Ser His Leu Val Glu Ala Leu Tyr Leu Val Cys Gly Glu Arg Gly Phe Phe Tyr Thr Pro Lys Thr
(SEQ ID NO: 11).

58. A pharmaceutical preparation comprising at least one insulin-zinc complex as claimed in one or more of claims 54 to 57.

59. The pharmaceutical preparation as claimed in claim 58, comprising an acidic solution.

60. The pharmaceutical preparation as claimed in claim 59 comprising an acidic solution of the insulin analog and/or the physiologically tolerable salt thereof as claimed in one or more of claims 1 to 21 with an appropriate amount of zinc ions, which makes possible the formation of an insulin-zinc complex as claimed in claim 54.

61. The pharmaceutical preparation as claimed in claim 59, comprising an insulin-zinc complex as claimed in one of claims 56 or 57.

62. The pharmaceutical preparation as claimed in one or more of claims 58 to 61, which contains the insulin-zinc complex in dissolved, amorphous and/or crystalline form.

63. An injectable solution having insulin activity, comprising the pharmaceutical preparation as claimed in claim 62 in dissolved form.

64. The injectable solution as claimed in claim 63, containing from 1 µg to 2 mg of zinc per ml.

65. The injectable solution as claimed in claim 64, containing from 5 µg to 200 µg of zinc per ml.

66. The use of the insulin-zinc complex as claimed in one or more of claims 54 to 57 for the production of a pharmaceutical preparation which has an insulin activity having a delayed onset of action.

## Revendications

1. Analogue d'insuline ou sel physiologiquement acceptable de celui-ci, de formule I 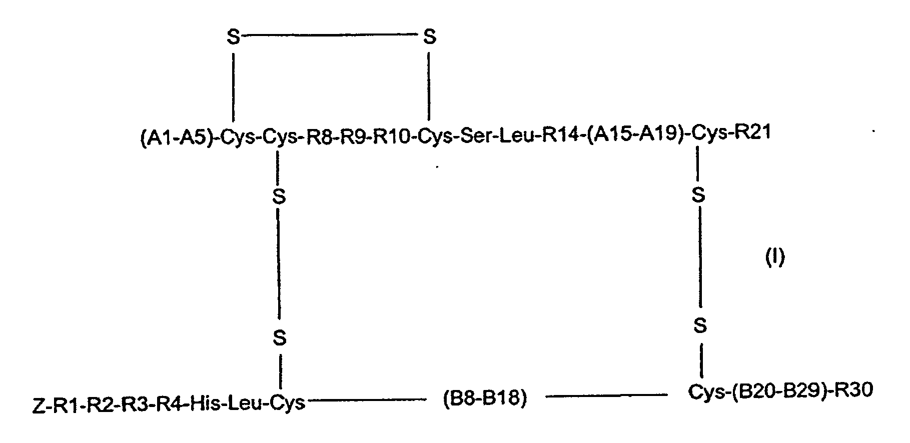 dans laquelle
| | |
|---|---|
| (A1-A5) | représentent les résidus aminoacide en les positions A1 à A5 de la chaîne A de l'insuline humaine ou d'une insuline animale, |
| (A15-A19) | représentent les résidus aminoacide en les positions A15 à A19 de la chaîne A de l'insuline humaine ou d'une insuline animale, |
| (B8-B18) | représentent les résidus aminoacide en les positions B8 à B18 de la chaîne B de |
| | l'insuline humaine ou d'une insuline animale, |
| (B20-B29) | représentent les résidus aminoacide en les positions B20 à B29 de la chaîne B de l'insuline humaine ou d'une insuline animale, |
| R8 | représente Thr ou Ala, |
| R9 | représente Ser ou Gly, |
| R10 | représente Ile ou Val, |
| R14 | représente Tyr, His, Asp ou Glu, |
| R21 | représente Asn, Asp, Gly, Ser, Thr, Ala, Glu ou Gln, |
| R1 | représente un résidu aminoacide quelconque génétiquement codable, est absent ou représente un atome d'hydrogène, |
| R2 | représente Val, Ala ou Gly, |
| R3 | représente Asn, His, Glu ou Asp, |
| R4 | représente Ala, Ser, Thr, Asn, Asp, Gln, Gly ou Glu, |
| R30 | représente un résidu aminoacide quelconque génétiquement codable ou -OH, |
| Z | représente un reste peptidique qui comporte de 1 à 4 résidus aminoacide génétiquement codable, contenant 1 à 4 résidus histidine (His), |
étant entendu que l'analogue d'insuline ou le sel physiologiquement acceptable de celui-ci, de formule I, ne se distingue pas seulement de l'insuline humaine par la variation des résidus aminoacide en les positions R3 ou R3 en conjonction avec R21 ou R3 en conjonction avec R4 dans la formule I.

2. Analogue d'insuline ou sel physiologiquement acceptable de celui-ci selon la revendication 1, **caractérisé en ce que** R8 représente Thr, R9 représente Ser et R10 représente Ile.

3. Analogue d'insuline ou sel physiologiquement acceptable de celui-ci selon la revendication 1 ou 2, **caractérisé en ce que** R1 représente Phe, His, Asn, Asp ou Gly.

4. Analogue d'insuline ou sel physiologiquement acceptable de celui-ci selon une ou plusieurs des revendications 1 à 3, **caractérisé en ce que** R30 représente Thr, Ala ou Ser.

5. Analogue d'insuline ou sel physiologiquement acceptable de celui-ci selon une ou plusieurs des revendications 1 à 4, **caractérisé en ce que** R21 représente Asn et R1 représente Phe.

6. Analogue d'insuline ou sel physiologiquement acceptable de celui-ci selon une ou plusieurs des revendications 1 à 5, **caractérisé en ce que** R2 représente Val, R3 représente Asn et R4 représente Gln.

7. Analogue d'insuline ou sel physiologiquement acceptable de celui-ci selon une ou plusieurs des revendications 1 à 6, **caractérisé en ce que** R14 représente Tyr.

8. Analogue d'insuline ou sel physiologiquement acceptable de celui-ci selon une ou plusieurs des revendications 1 à 6, **caractérisé en ce que** R14 représente His.

9. Analogue d'insuline ou sel physiologiquement acceptable de celui-ci selon une ou plusieurs des revendications 1 à 6, **caractérisé en ce que** R14 représente Asp.

10. Analogue d'insuline ou sel physiologiquement acceptable de celui-ci selon une ou plusieurs des revendications 1 à 6, **caractérisé en ce que** R14 représente Glu.

11. Analogue d'insuline ou sel physiologiquement acceptable de celui-ci selon une ou plusieurs des revendications 1 à 6, **caractérisé en ce que** R30 représente Thr.

12. Analogue d'insuline ou sel physiologiquement acceptable de celui-ci selon une ou plusieurs des revendications 1 à 10, **caractérisé en ce que** R30 représente Ala.

13. Analogue d'insuline ou sel physiologiquement acceptable de celui-ci selon une ou plusieurs des revendications 1 à 10, **caractérisé en ce que** R30 représente Ser.

14. Analogue d'insuline ou sel physiologiquement acceptable de celui-ci selon une ou plusieurs des revendications 1 à 3 et 5 à 10, **caractérisé en ce que** R30 représente -OH.

15. Analogue d'insuline ou sel physiologiquement acceptable de celui-ci selon une ou plusieurs des revendications 1 à 14, **caractérisé en ce que** Z représente His.

16. Analogue d'insuline ou sel physiologiquement acceptable de celui-ci selon une ou plusieurs des revendications 1 à 14, **caractérisé en ce que** Z représente His-Ala.

17. Analogue d'insuline ou sel physiologiquement acceptable de celui-ci selon une ou plusieurs des revendications 1 à 14, **caractérisé en ce que** Z représente His-Ala-Ala.

18. Analogue d'insuline ou sel physiologiquement acceptable de celui-ci selon la revendication 15, **caractérisé en ce que** la chaîne B présente la séquence
His Phe Val Asn Gln His Leu Cys Gly Ser His Leu Val Glu Ala Leu Tyr Leu Val Cys Gly Glu Arg Gly Phe Phe Tyr Thr Pro Lys
(SEQ ID n° 3).

19. Analogue d'insuline ou sel physiologiquement acceptable de celui-ci selon la revendication 15, **caractérisé en ce que** la chaîne B présente la séquence
His Phe Val Asn Gln His Leu Cys Gly Ser His Leu Val Glu Ala Leu Tyr Leu Val Cys Gly Glu Arg Gly Phe Phe Tyr Thr Pro Lys Thr
(SEQ ID n° 4).

20. Analogue d'insuline ou sel physiologiquement acceptable de celui-ci selon la revendication 16, **caractérisé en ce que** la chaîne B présente la séquence
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
(SEQ ID n° 5).

21. Analogue d'insuline ou sel physiologiquement acceptable de celui-ci selon la revendication 17, **caractérisé en ce que** la chaîne B présente la séquence
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr.
(SEQ ID n° 6).

22. Procédé pour la préparation d'un analogue d'insuline ou d'un sel physiologiquement acceptable de celui-ci selon une ou plusieurs des revendications 1 à 21, comprenant la construction d'un véhicule d'expression réplicable qui contient une séquence d'ADN codant pour un précurseur de l'analogue d'insuline ayant la séquence générale d'aminoacides II
Met-X²ₘ- (Arg)ₚ-Z-R1-R2-R3-R4-His-Leu-Cys-(B8-B18)-Cys-(B20-B29)-R30-X¹ₙ-Arg-(A1-A5)-Cys-Cys-R8-Ser-R10-Cys-Ser-Leu-R14-(A15-A19)-Cys-R21 II,
dans laquelle
| | |
|---|---|
| X¹ₙ | représente une chaîne peptidique comportant n résidus aminoacide, n représentant un nombre entier allant de 0 à 34, |
| X²ₘ | représente une chaîne peptidique comportant m résidus aminoacide, m représentant un nombre entier allant de 0 à 20, |
| p | est 0, 1 ou 2, |
| R30 | représente un résidu aminoacide quelconque génétiquement codable ou est absent et |
| Z | représente un reste peptidique comportant 1 à 4 résidus aminoacide génétiquement codables, comportant 1 à 4 résidus histidine (His) |
et les autres variables ont les significations données dans la revendication 1, les conditions mentionnées dans la revendication 1 étant également valables, l'expression dans une cellule hôte et la libération de l'analogue d'insuline à partir de son précurseur, à l'aide de méthodes chimiques et/ou enzymatiques.

23. Procédé selon la revendication 22, **caractérisé en ce que** la cellule hôte est une bactérie.

24. Procédé selon la revendication 23, **caractérisé en ce que** la bactérie est *E*. *coli.*

25. Procédé selon la revendication 22, **caractérisé en ce que** la cellule hôte est une levure.

26. Procédé selon la revendication 25, **caractérisé en ce que** la levure est *Saccharomyces cerevisiae.*

27. Procédé selon l'une quelconque des revendications 22 à 26, pour la préparation d'un analogue d'insuline selon la revendication 19, **caractérisé en ce que** le précurseur de l'analogue d'insuline présente la séquence
Met Ala Thr Thr Ser Thr Gly Asn Ser Ala Arg
His Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys Ser Leu Tyr Gln Leu Glu Asn Tyr Cys Asn
(SEQ ID n° 7).

28. Procédé selon l'une quelconque des revendications 22 à 26, pour la préparation d'un analogue d'insuline selon la revendication 20, **caractérisé en ce que** le précurseur de l'analogue d'insuline présente la séquence
Met Ala Thr Thr Ser Thr Gly Asn Ser Ala Arg
His Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys Ser Leu Tyr Gln Leu Glu Asn Tyr Cys Asn
(SEQ ID n° 8).

29. Procédé selon l'une quelconque des revendications 22 à 26, pour la préparation d'un analogue d'insuline selon la revendication 21, **caractérisé en ce que** le précurseur de l'analogue d'insuline présente la séquence
Met Ala Thr Thr Ser Thr Gly Asn Ser Ala Arg
His Ala Ala Phe Val Asn Gln His Leu Cys Gly Ser His Leu Val Glu Ala Leu Tyr Leu Val Cys Gly Glu Arg Gly Phe Phe Tyr Thr Pro Lys Thr
Arg Arg Glu Ala Glu Asp Pro Gln Val Gly Gln Val Glu Leu Gly Gly Gly Pro Gly Ala Gly Ser Leu Gln Pro Leu Ala Leu Glu Gly Ser Leu Gln Lys Arg
Gly Ile Val Glu Gln Cys Cys Thr Ser Ile Cys Ser Leu Tyr Gln Leu Glu Asn Tyr Cys Asn
(SEQ ID n° 9).

30. Précurseur de l'analogue d'insuline selon la revendication 22.

31. Précurseur de l'analogue d'insuline selon la revendication 27.

32. Précurseur de l'analogue d'insuline selon la revendication 28.

33. Précurseur de l'analogue d'insuline selon la revendication 29.

34. Séquence d'ADN qui code pour un précurseur de l'analogue d'insuline selon la revendication 30.

35. Séquence d'ADN qui code pour un précurseur de l'analogue d'insuline selon la revendication 31.

36. Séquence d'ADN qui code pour un précurseur de l'analogue d'insuline selon la revendication 32.

37. Séquence d'ADN qui code pour un précurseur de l'analogue d'insuline selon la revendication 33.

38. Vecteur d'expression contenant une séquence d'ADN selon la revendication 34.

39. Vecteur d'expression contenant une séquence d'ADN selon la revendication 35.

40. Vecteur d'expression contenant une séquence d'ADN selon la revendication 36.

41. Vecteur d'expression contenant une séquence d'ADN selon la revendication 37.

42. Cellule hôte isolée, qui est transformée par un véhicule d'expression selon l'une quelconque des revendications 38 à 41.

43. Préparation pharmaceutique contenant au moins un analogue d'insuline et/ou au moins un sel physiologiquement acceptable de celui-ci selon une ou plusieurs des revendications 1 à 21.

44. Préparation pharmaceutique selon la revendication 43, **caractérisée en ce qu'**elle contient l'analogue d'insuline et/ou le sel physiologiquement acceptable de celui-ci, sous forme dissoute, amorphe et/ou cristalline.

45. Préparation pharmaceutique selon la revendication 43 ou 44, **caractérisée en ce qu'**elle contient en outre un adjuvant retard.

46. Préparation pharmaceutique selon la revendication 45, **caractérisée en ce que** l'adjuvant retard est le sulfate de protamine, l'analogue d'insuline et/ou le sel physiologiquement acceptable de celui-ci se trouvant en un produit de co-cristallisation avec le sulfate de protamine.

47. Préparation pharmaceutique selon une ou plusieurs des revendications 43 à 46, **caractérisée par** une teneur supplémentaire en insuline humaine non modifiée.

48. Préparation pharmaceutique selon une ou plusieurs des revendications 43 à 47, **caractérisée par** une teneur supplémentaire en un autre analogue d'insuline.

49. Préparation pharmaceutique selon la revendication 48, **caractérisée en ce que** l'autre analogue d'insuline est la Gly(A21)-Arg(B31)-Arg(B32)-insuline humaine.

50. Solution injectable à activité d'insuline, contenant la préparation pharmaceutique selon une ou plusieurs des revendications 43 à 49, sous forme dissoute.

51. Solution injectable selon la revendication 50,
**caractérisée par** une teneur en zinc de 1 *µ*g à 2 mg par ml.

52. Solution injectable selon la revendication 50, **caractérisée par** une teneur en zinc de 5 *µ*g à 200 *µ*g par ml.

53. Utilisation de l'analogue d'insuline et/ou d'un sel physiologiquement acceptable de celui-ci selon une ou plusieurs des revendications 1 à 21, pour la fabrication d'une préparation pharmaceutique qui présente une activité d'insuline à apparition retardée de l'effet.

54. Complexe insuline-zinc contenant un hexamère d'insuline et 4 à 10 ions zinc par hexamère d'insuline, **caractérisé en ce que** l'hexamère d'insuline consiste en six molécules d'un analogue d'insuline de formule I selon une ou plusieurs des revendications 1 à 21.

55. Complexe insuline-zinc selon la revendication 54, contenant 5 à 8 ions zinc par hexamère d'insuline.

56. Complexe insuline-zinc selon la revendication 54 ou 55, **caractérisé en ce que** la chaîne B de l'analogue d'insuline de formule I présente la séquence
Phe Val His Gln His Leu Cys Gly Ser His Leu Val Glu Ala Leu Tyr Leu Val Cys Gly Glu Arg Gly Phe Phe Tyr Thr Pro Lys Thr
(SEQ ID n° 10).

57. Complexe insuline-zinc selon la revendication 54 ou 55, **caractérisé en ce que** la chaîne B de l'analogue d'insuline de formule I présente la séquence
Phe Val Asp Gln His Leu Cys Gly Ser His Leu Val Glu Ala Leu Tyr Leu Val Cys Gly Glu Arg Gly Phe Phe Tyr Thr Pro Lys Thr
(SEQ ID n° 11).

58. Préparation pharmaceutique contenant au moins un complexe insuline-zinc selon une ou plusieurs des revendications 54 à 57.

59. Préparation pharmaceutique selon la revendication 58, contenant une solution acide.

60. Préparation pharmaceutique selon la revendication 59, contenant une solution acide de l'analogue d'insuline et/ou du sel physiologiquement acceptable de celui-ci selon une ou plusieurs des revendications 1 à 21, avec une quantité correspondante d'ions zinc, permettant la formation d'un complexe insuline-zinc selon la revendication 54.

61. Préparation pharmaceutique selon la revendication 59, contenant un complexe insuline-zinc selon la revendication 56 ou 57.

62. Préparation pharmaceutique selon une ou plusieurs des revendications 58 à 61, **caractérisée en ce qu'**elle contient le complexe insuline-zinc sous forme dissoute, amorphe et/ou cristalline.

63. Solution injectable à activité d'insuline, contenant la préparation pharmaceutique selon la revendication 62, sous forme dissoute.

64. Solution injectable selon la revendication 63, **caractérisée par** une teneur en zinc de 1 *µ*g à 2 mg par ml.

65. Solution injectable selon la revendication 64, **caractérisée par** une teneur en zinc de 5 *µ*g à 200 *µ*g par ml.

66. Utilisation du complexe insuline-zinc selon une ou plusieurs des revendications 54 à 57, pour la fabrication d'une préparation pharmaceutique présentant une activité d'insuline à apparition retardée de l'effet.
